# EUROPEAN PATENT APPLICATION

(11) **EP 2 347 732 A1**
(43) Date of publication of application: **27.07.2011**
(21) Application number: 11150578.0
(22) Date of filing: 11.01.2011
(51) Int. Cl.: A61F 2/38

(54) **Knee prosthesis system**

(30) Priority: 21.01.2010 US 691280
(71) Applicant: DePuy Products, Inc., Warsaw, IN 46581 (US)
(72) Inventor: Deffenbaugh, Daren L, Winona Lake, IN 46590 (US); Hazebrouck, Stephen A, Winona Lake, IN 46590 (US)
(74) Representative: Belcher, Simon James

(57) **Abstract**

A knee prosthesis system (100) for total knee replacement of a patient has a plurality of distinctly-sized fixed tibial components (70), each of which has a fixed platform (72) defining a first, bone covering profile and a plurality of distinctly-sized mobile tibial components (80), each of which has a mobile platform (82) defining a second, bone covering profile. The first and second, bone covering profiles are approximately the same for any one of the plurality of distinctly-sized fixed tibial components and a correspondingly-sized one of the plurality of distinctly-sized mobile tibial components.

## Description

This invention relates to knee prosthesis systems.

The most widely-used type of knee prosthesis for implantation into a patient during a total knee replacement (TKR) procedure includes three components: a metallic, femoral component that attaches to the distal femur; a metallic, tibial component (or tray) that attaches to the proximal tibia; and a polymeric (UHMWPE), insert (also called a bearing or an inlay) that fits between the femoral and tibial components. Various types of patella replacements are also available for use in combination with some of these knee prostheses. Two types of knee prostheses are a posterior-stabilized (PS) prosthesis, for when the posterior cruciate ligament is no longer viable, and a (posterior) cruciate-retaining (CR) knee prosthesis. Each of these two types of knee prostheses may be provided as a fixed bearing knee prosthesis, in which the insert does not move relative to the tibial component, or a mobile bearing knee prosthesis, in which the insert rotates upon a smooth platform of the tibial component. Whether to use a mobile insert or a fixed insert depends largely on the condition of the patient's knee ligaments and other soft tissues.

A knee prosthesis system may include numerous sizes of femoral, tibial and insert components to accommodate the variation of patient anatomies in the worldwide TKR patient population. The design of a knee prosthesis system requires trade-offs among many important factors related to kinematic performance, clinical outcomes, implant longevity, cost, and ease of use, to name just a few. An important consideration relative to both the kinematic performance and the life of the knee prosthesis is the degree of conformity between the femoral component bearing surfaces and the insert bearing surfaces.

US-7628818 discloses a fixed bearing, knee prosthesis system in which each of differently sized inserts are compatible with each size of tibial component, so that it is possible for a surgeon to select a tibial component that is properly sized for a patient's tibia, and an insert that is matched with the femoral component.

US-A-2009/0326666 discloses a knee prosthesis system having a plurality of distinctly-sized PS inserts (fixed or mobile) having a spine extending superiorly from an inferior surface. The spine has a posterior side that has a concave cam surface and a convex cam surface. Each of the PS femoral components has a pair of spaced-apart condyles defining an intracondylar notch that has a posterior cam. The posterior cam includes a concave cam surface and a convex cam surface. The concave cam surface of the posterior cam contacts the convex cam surface of the spine during a first range of flexion and the convex cam surface of the posterior cam contacts the concave cam surface of the spine during a second range of flexion.

Investigators typically characterize conformity in either the coronal plane or sagittal plane as the ratio of the convex radius of a femoral condyle of the femoral component to the concave radius of the interfacing insert surface. A conformity ratio of zero represents a flat insert surface, corresponding to very high contact stress at high loads. A conformity ratio of 0.99 represents high conformity, corresponding, in general, to high contact area, relatively low contact stress and, subsequently, reduced wear rate of the polyethylene surface of the insert.

Investigators have found that conformity in the coronal plane may affect prosthesis life more than conformity in the sagittal plane. For example, in an article by Kuster, et al, "The effects of conformity and load in total knee replacement" (Clinical Orthopaedics and Related Research, Number 375, pp. 302-12, June 2000), the authors found that the compressive surface stress, the shear stress and the von Mises stress were affected by changes to the conformity ratio and to a lesser extent by load changes. In a more recent article by Berend, et al, "Effects of coronal plane conformity on tibial loading in TKA: a comparison of AGC flat versus conforming articulations" (Surgical Technology Int., Number 18, pp. 207-212, 2009), the authors studied the effect of conformity on loading of the proximal tibia of the patient. Improper loading of the proximal tibia may lead to aseptic loosening of the tibial component in the tibia and eventually prosthesis failure requiring revision surgery. The authors found that coronally dished components created a strain increase in the anterior medial tibia while creating a significant strain decrease in the posterior tibia. They also found that proximal tibial strains were decreased and centralized in conforming versus flat articulations.

It is known in the art, however, that very high conformity may also lead, for example, to undesirable loading conditions on the insert surface or to excessive constraint of the femoral component, thereby inhibiting joint motions important to joint performance and patient comfort. Therefore, designs with intermediate values of contact area may be optimal as long as the stresses are below the yield strength of the insert material, in order to provide the optimal combination of joint laxity and conformity.

Complicating the challenge faced by knee prosthesis designers is the variability of patient anatomies in the worldwide, TKR patient population. Smaller patients with smaller femurs require, obviously, smaller knee prostheses. Each of the medial and lateral condyles of a femoral component of a small femoral component has a smaller coronal radius than a large femoral component for a large patient. To maintain the appropriate comformity ratio, as well as other geometrical relationships including condylar spacing, the small femoral component must be matched to a properly sized insert. However, in addition to the wide range of patient sizes, the dimensional proportionality between the femur and tibia bones also varies widely. For example, some patients have a larger distal femur than other patients for a given size of the proximal tibia. In such cases when using currently available knee prosthesis systems, the surgeon may need to choose to implant a femoral component that is slightly mismatched with the femur and matched with the insert, or a femoral component that is matched with the femur and slightly mismatched with the insert.

The present invention provides a knee prosthesis system from which a surgeon may select a fixed or a mobile, total knee replacement prosthesis for implantation into a patient, the knee prosthesis system comprising:
a plurality of distinctly-sized fixed tibial components, each of which has a fixed platform defining a first, bone covering profile;
a plurality of distinctly-sized mobile tibial components, each of which has a mobile platform defining a second, bone covering profile;
in which the first and second, bone covering profiles are approximately the same for any one of the plurality of distinctly-sized fixed tibial components and a correspondingly-sized one of the plurality of distinctly-sized mobile tibial components.

Optionally, the knee prosthesis system includes a plurality of distinctly-sized femoral components.

Optionally, the knee prosthesis system includes a plurality of fixed inserts, each of which is sized and shaped for implantation between one of the plurality of distinctly-sized femoral components and at least one of the plurality of distinctly-sized fixed tibial components.

Optionally, the knee prosthesis system includes a plurality of mobile inserts, each of which is sized and shaped for implantation between one of the plurality of distinctly-sized femoral components and at least one of the plurality of distinctly-sized mobile tibial components.

Optionally, each of the plurality of distinctly-sized fixed tibial components further includes a fixed stem extending distally from the fixed platform, and each of the plurality of distinctly-sized mobile tibial components further includes a mobile stem extending distally from the mobile platform. Optionally, the size and shape of the fixed stem of each of the plurality of distinctly-sized fixed tibial components is approximately the same as the size and shape of the mobile stem of a correspondingly-sized one of the plurality of distinctly-sized mobile tibial components.

Optionally, each of the fixed stems and each of the mobile stems include a distal portion having a distal length and a proximal portion having any one of a plurality of proximal lengths, such that each of the fixed stems and each of the mobile stems may have any one of a plurality of overall stem lengths.

Optionally, the distal portion of each of the fixed stems and the distal portion of each of the mobile stems has an approximately conical shape.

Optionally, the proximal portion of each of the fixed stems and the proximal portion of each of the mobile stems has an approximately frustoconical shape.

The invention also provides a knee prosthesis system from which a surgeon may select a fixed or mobile, total knee replacement prosthesis for implantation into a patient, the knee prosthesis system comprising:
a plurality of distinctly-sized fixed tibial components, each of which has a fixed platform defining a first, bone covering profile;
a plurality of distinctly-sized mobile tibial components, each of which has a mobile platform defining a second, bone covering profile;
a plurality of distinctly-sized femoral components;
a plurality of fixed inserts, each of which is sized and shaped for implantation between one of the plurality of distinctly-sized femoral components and at least one of the plurality of distinctly-sized fixed tibial components; and
a plurality of mobile inserts, each of which is sized and shaped for implantation between one of the plurality of distinctly-sized femoral components and at least one of the plurality of distinctly-sized mobile tibial components;
in which the first and second, bone covering profiles are approximately the same for any one of the plurality of distinctly-sized fixed tibial components and a correspondingly-sized one of the plurality of distinctly-sized mobile tibial components.

Optionally, each of the plurality of distinctly-sized fixed tibial components further includes a fixed stem extending distally from the fixed platform, and each of the plurality of distinctly-sized mobile tibial components further includes a mobile stem extending distally from the mobile platform. Optionally, the size and shape of the fixed stem of each of the plurality of distinctly-sized fixed tibial components is approximately the same as the size and shape of the mobile stem of a correspondingly-sized one of the plurality of distinctly-sized mobile tibial components.

Optionally, each of the fixed stems and each of the mobile stems include a distal portion having a distal length and a proximal portion having any one of a plurality of proximal lengths, such that each of the fixed stems and each of the mobile stems may have any one of a plurality of overall stem lengths.

Optionally, the distal portion of each of the fixed stems and the distal portion of each of the mobile stems has an approximately conical shape.

Optionally, the proximal portion of each of the fixed stems and the proximal portion of each of the mobile stems has an approximately frustoconical shape.

It would be preferable for a surgeon to be able to select a femoral component that is sized to fit the femur of a particular patient, a tibial component that is sized to fit the tibia, and an insert that optimally matches the femoral component and is compatible with the tibial component. Such a knee prosthesis system should include both fixed and mobile types of prostheses and provide for both CR and PS procedures. Furthermore, the system should accommodate the wide variety of patient anatomies in the worldwide population.

It is desirable to maintain or lower the costs and complexity of knee prosthesis systems. A knee prosthesis system may include femoral, tibial and insert components in a number of sizes, for each of the right and left knees, to accommodate variations in patient anatomies and conditions. In addition, each of inserts may be provided in a number of thicknesses so that the surgeon may select the one that results in the appropriate joint tension. Consequently, knee prosthesis manufacturers must provide a very large inventory of components representing a large number of different size combinations to accommodate the worldwide patient population. What is needed, therefore, is an improved, knee prosthesis system that allows component interchangeability to provide the necessary size combinations with a minimal number of components.

Another consideration during the design of knee prosthesis systems is bone preparation for implantation of the PS femoral component. Both the PS and the CR femoral components have a pair of spaced-apart condyles that are somewhat similar to the natural condyles of the distal femur. For the PS femoral component, a box (or intracondylar notch) positioned between the condyles includes features for interaction with a spine on the PS insert. Implantation of the PS femoral component requires cutting a recess into the distal femur to receive the box. In some current, knee prosthesis systems, the size of the box is the same for all of the PS femoral component sizes, thereby requiring cutting the same size recess into the distal femur, even for smaller femurs. It is desirable, however, to conserve natural bone, if possible, during preparation of the femur for attachment of the femoral component. There is a further need, therefore, for a knee prosthesis system in which each of the PS femoral components has a box that is sized proportionately to the femur size, while also addressing the previously described needs.

Yet another consideration during the design of knee prosthesis systems is bone preparation for implantation of the tibial component. Currently available, mobile and fixed TKR prosthesis systems include tibial components for a range of anatomical sizes. For some of these systems, the tibial component for a mobile TKR prosthesis of a particular size has a different configuration than that of a fixed TKR prosthesis of the same size. Specifically, the platform that supports the fixed bearing insert may have a different shape than the platform that supports the mobile bearing insert. This may result in a small, but possibly significant, difference in coverage of the resected, tibial plateau surface. Although less than ideal, one way surgeons may obtain the desired, tibial bone coverage is to select a larger size tibial component. What is more desirable is a TKR system that has mobile and fixed tibial components with a common platform profile shape that is optimized for interaction with surrounding tissues, kinematic performance, etc.

Also, currently available TKR systems have tibial components with stems of variable lengths to accommodate different tibial bone conditions. Furthermore, the stems for mobile tibial components may have a different configuration than the stems for fixed tibial components. Subsequently, such systems require that a number of different reaming instruments be available for each surgical procedure. A preferable TKR system would have mobile and fixed tibial components with stems of different lengths, but not requiring several different reaming instruments for preparing the tibia. This would also provide the surgeon with the intraoperative flexibility to select the appropriate type of tibial component, while reducing the number of instruments that would need to be available during the surgical procedure.

Embodiments of the invention are described below by way of example with reference to the accompanying drawings, in which common reference numerals identify the common elements, and in which:
Fig. 1 is a perspective view of a fixed CR prosthesis 110.
Fig. 2 is a perspective view of a CR femoral component 20, which is part of fixed CR prosthesis 110 shown in Fig. 1 and mobile CR prosthesis 130 shown in Fig. 9.
Fig. 3 is a perspective view of a fixed CR insert 50, which is part of fixed CR prosthesis 110 shown in Fig. 1.
Fig. 4 is a perspective view of a fixed tibial component 70, which is part of fixed CR prosthesis 110 shown in Fig. 1 and a fixed PS prosthesis 120 shown in Fig. 5.
Fig. 5 is a perspective view of fixed PS prosthesis 120.
Fig. 6 is a perspective view of a PS femoral component 10, which is part of fixed PS prosthesis 120 shown in Fig. 5 and a mobile PS prosthesis 140 shown in Fig. 13.
Fig. 7 is a perspective view of a fixed PS insert 30, which is part of fixed PS prosthesis 120 shown in Fig. 5.
Fig. 8 is a perspective view of fixed tibial component 70, which is also shown in Fig. 4.
Fig. 9 is a perspective view of a mobile CR prosthesis 130.
Fig. 10 is a perspective view of CR femoral component 20, which is also shown in Fig. 2.
Fig. 11 is a perspective view of a mobile CR insert 60, which is part of mobile CR prosthesis 130 shown in Fig. 9.
Fig. 12 is a perspective view of a mobile tibial component 80, which is part of mobile CR prosthesis 130 and a mobile PS prosthesis 140 shown in Fig. 13.
Fig. 13 is a perspective view of mobile PS prosthesis 140.
Fig. 14 is a perspective view of PS femoral component 10, which is also shown in Fig. 10.
Fig. 15 is a perspective view of a mobile PS insert 40, which is part of mobile PS prosthesis 140 shown in Fig. 13.
Fig. 16 is a perspective view of mobile tibial component 80, which is also shown in Fig. 12.
Fig. 17 is a chart representing knee prosthesis system 100 for configuring, in a plurality of size combinations, each of the prostheses shown in Figs. 1, 5, 9 and 13.
Fig. 18 is an anterior view of a size one, mobile tibial component.
Fig. 19 is an anterior view of a size three, mobile tibial component.
Fig. 20 is an anterior view of a size seven, mobile tibial component.
Fig. 21 is an anterior view of a size nine, mobile tibial component.
Fig. 22A is a superior view of the size one, mobile tibial component of Fig. 18.
Fig. 22B is a superior view of a size one, fixed tibial component.
Fig. 23A is a superior view of the size three, mobile tibial component of Fig. 19.
Fig. 23B is a superior view of a size three, fixed tibial component.
Fig. 24A is a superior view of the size seven, mobile tibial component of Fig. 20.
Fig. 24B is a superior view of a size seven, fixed tibial component.
Fig. 25A is a superior view of the size nine, mobile tibial component of Fig. 21.
Fig. 25B is a superior view of a size nine, fixed tibial component.

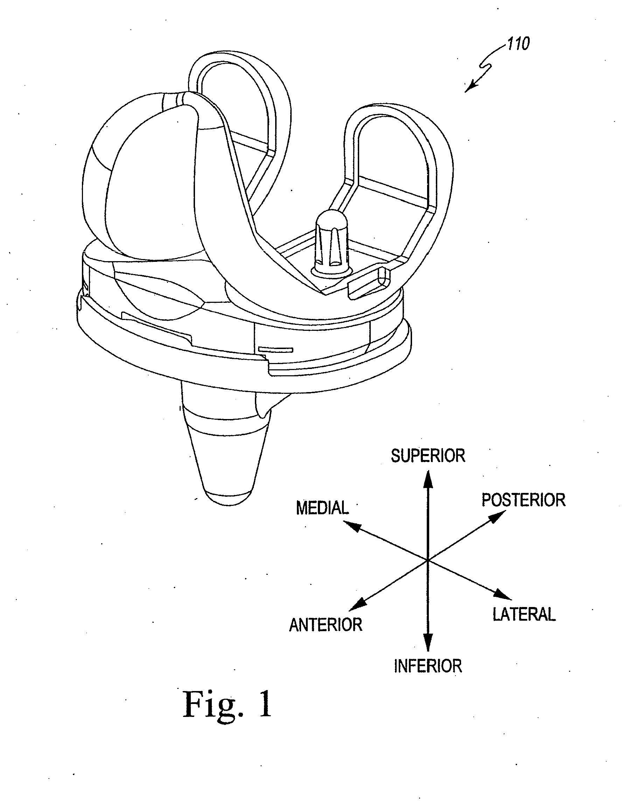

In this document, the terms "anterior, posterior, lateral, medial" generally refer to the front, back, outside and midline of the surgical patient, respectively, although we also use these terms in reference to the devices. Fig. 1 shows directional arrows for these terms and the terms "inferior, superior". Also, references to "surgeon" and "user" are applicable to any person who might assist a surgeon during the surgical procedure.

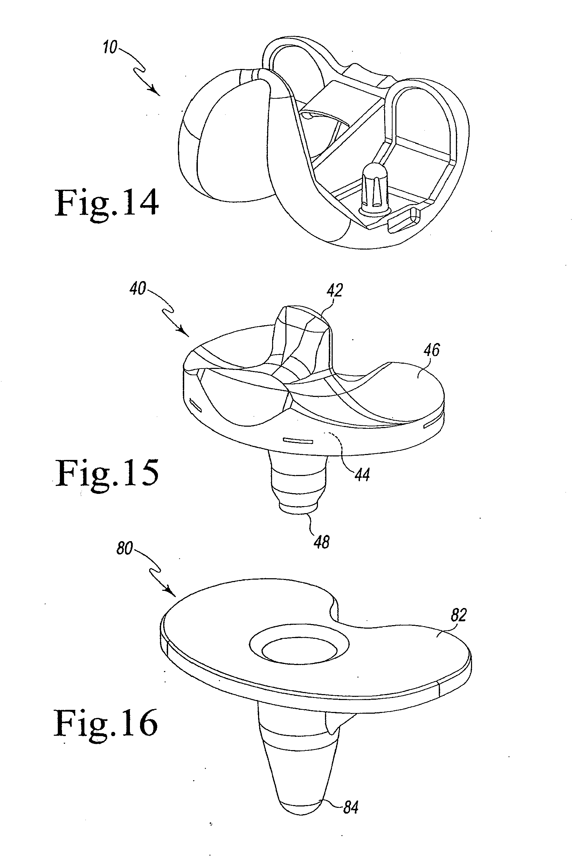

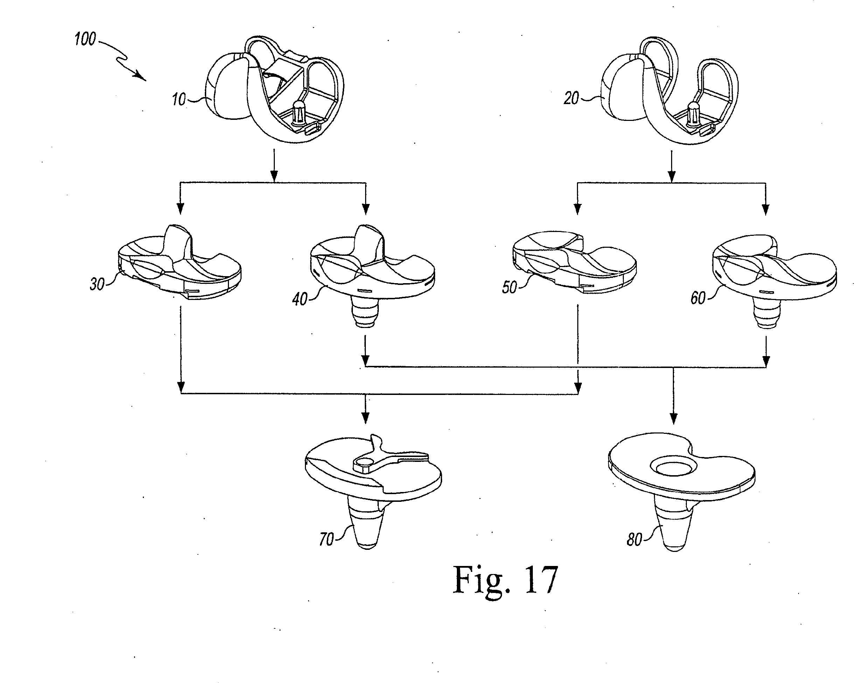

Referring to the drawings, Figs. 1 to 16 show components of a knee prosthesis system 100 that is shown in Fig. 17. Each of these components may be provided in a plurality of sizes and may be matched together as described next, thereby providing the surgeon with a large plurality of size combinations. Using knee prosthesis system 100, the surgeon may select, for each patient in a large patient population, a size combination that correctly matches both the femur and the tibia of the patient. That is, the femoral component is distinctly-sized to fit the femur and the tibial component is distinctly-sized to fit the tibia, and the components of the knee prosthesis are optimally match to avoid compromising joint performance.

One characteristic of the distinctly-sized femoral and tibial components of knee prosthesis system 100 is proportionality of each component to the particular size of bone to which the component is to be attached. In general, the dimensional scale of the component varies, but not the shape. For example, the femoral component may have a proportionally-sized, intercondylar distance, such that a large femoral component has a proportionally longer intercondylar distance than that of a small femoral component. Similarly, a large tibial component may have a proportionally wider and deeper, posterior notch than that of a small tibial component.

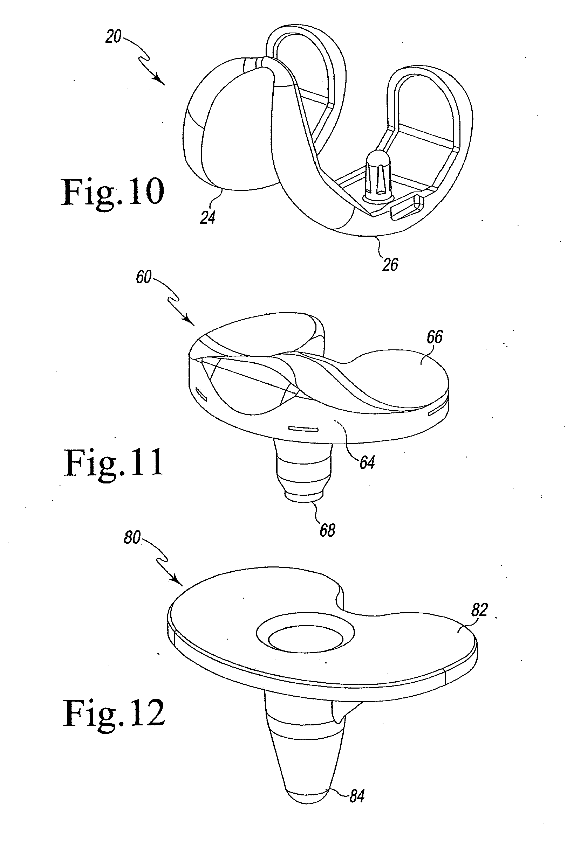

Fig. 1 is a perspective view of a cruciate-retaining, fixed bearing, knee prosthesis 110, also referred to as a fixed CR prosthesis 110, which includes a CR femoral component 20 (Fig. 10), a fixed CR insert 50 (Fig. 11) and a fixed tibial component 70 (Fig. 12). Fixed CR prosthesis 110 may be identical to or similar to the knee prosthesis shown in Fig. 1 of US-7628818. Any one of a plurality of differently-sized inserts (or bearings) may be secured to any one of a plurality of differently-sized tibial components (or trays). As a result, articulation surface geometries and other features of the insert may be enhanced for each size of femoral component. Such interchangeability also allows for smaller size increments in the design of a range of femoral components. CR femoral component 20 includes a medial condyle 24 and a lateral condyle 26, both of which articulate upon a superior surface 56 of fixed CR bearing 50. An anterior buttress 75 and a posterior buttress 76 of fixed tibial component 70 fixedly retain CR insert 50 to fixed tibial component 70, such that an inferior surface 54 of CR insert 50 rests on a platform 72 of fixed tibial component 70. Fixed tibial component 70 also includes a stem 74 that inserts into the surgically prepared proximal tibia.

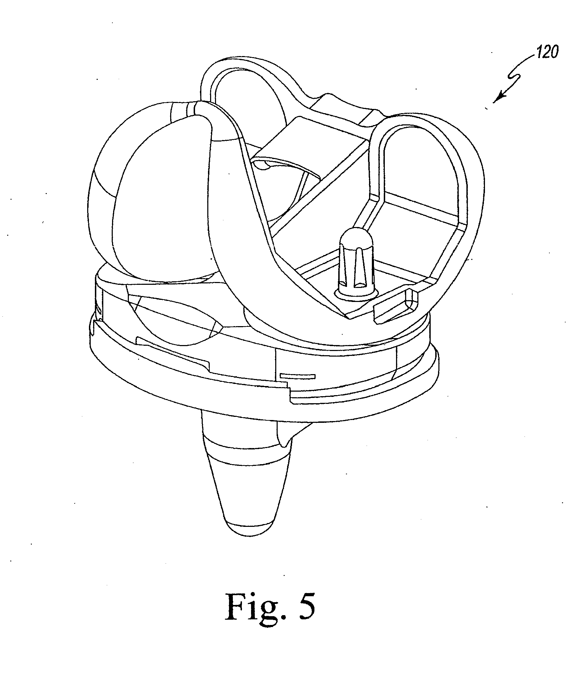

Fig. 5 is a perspective view of a posterior-stabilized, fixed bearing, knee prosthesis 120, also referred to as a fixed PS prosthesis 120, which includes a PS femoral component 10, a fixed PS insert 30 and fixed tibial component 70. PS femoral component 10 may be identical to or similar to the femoral component shown in Fig. 1 of US-A-2009/0326666. PS femoral component 10 includes a medial condyle 14 and a lateral condyle 16, both of which articulate upon a superior surface 36 of fixed PS insert 30. PS femoral component 10 also includes a box 32 positioned between medial condyle 14 and lateral condyle 16. Box 32 encases a posterior cam and an anterior cam (both hidden) that operationally engage with a spine 32 of fixed PS insert 30 as described in US-A-2009/0326666. Anterior buttress 75 and posterior buttress 776 of fixed tibial component 70 retain PS insert 30, such that an inferior surface 34 of PS insert 30 rests on a platform 72 of fixed tibial component 70.

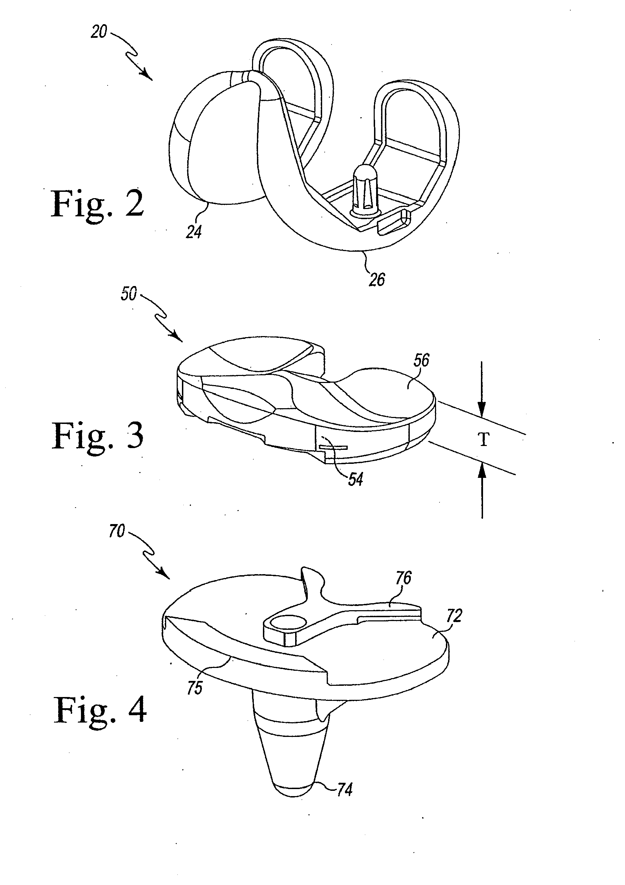

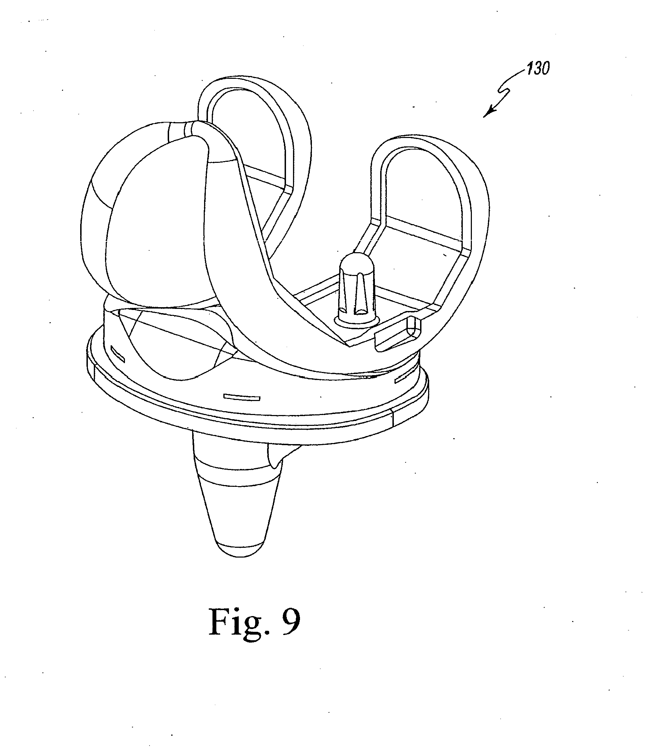

Fig. 9 is a perspective view of a cruciate-retaining, mobile bearing, knee prosthesis 130, also referred to as a mobile CR prosthesis 130, which includes CR femoral component 20 (previously described for Fig. 2), a mobile CR bearing insert 60 and a mobile tibial component 80. Medial condyle 24 and lateral condyle 26 of CR femoral component 20 articulate on a superior surface 66 of mobile CR insert 60. An inferior surface 64 of mobile CR insert 60 articulates against platform 82 of mobile tibial component 80. A post 68 extends inferiorly from inferior surface 64 and rotatably inserts into a hollow stem 84 of mobile tibial component 80.

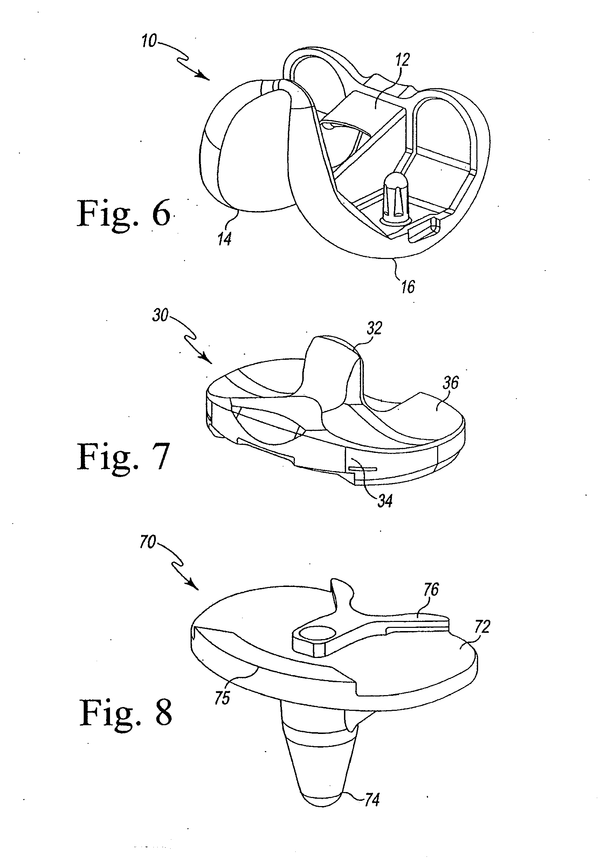

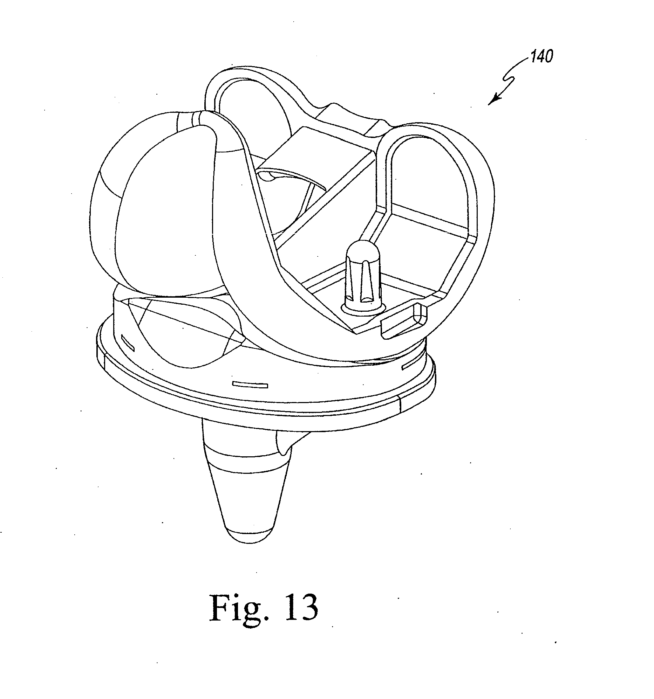

Fig. 13 is a perspective view of a posterior-stabilized, mobile bearing, knee prosthesis 140, also referred to as a mobile PS prosthesis 140, which includes PS femoral component 10 (previously described for Fig. 6), a mobile PS insert 40 and mobile tibial component 80 (previously described for Fig. 12). Mobile PS insert 40 includes a superior surface 46 and a spine 42 that may be identical to superior surface 36 and spine 32 of fixed PS insert 30 shown in Fig. 7. Mobile PS insert 40 also includes an inferior surface 34 that may be identical to inferior surface 54 of fixed CR insert 50 shown in Fig. 3.

Fig. 17 is a chart representing an integrated, knee prosthesis system 40 that includes each of the knee prostheses shown in Figs. 1, 5, 9 and 13. Each of components 10, 20, 30, 40, 50, 60, 70 and 80 may be provided in a plurality of sizes (for example, ten sizes) to accommodate the wide variation of anatomies of the patient population. These components may be matched together as follows:
Any one size of PS femoral component 10 may be matched with any one size of either fixed PS insert 30 or mobile insert 40.
Any one size of CR femoral component 20 may be matched with any one size of either fixed CR insert 50 or mobile CR insert 60.
Any one size of fixed tibial component 70 may be matched with any one size of either fixed PS insert 30 or fixed CR insert 50.
Any one size of mobile tibial component 80 may be matched with any one size of either mobile PS insert 40 or mobile CR insert 60.

In addition, each size of each of inserts 30, 40, 50 and 60 may be provided in a plurality of thicknesses.

As noted earlier, the anatomies of patients vary not only in size, but also in femur/tibia, size proportionality. Using historical data for TKR procedures, it is possible to determine the size combinations that would be needed for the majority of patients in the worldwide population. For example, each of practically all patients may be accommodated with a knee prosthesis distinctly-sized to fit both the femur and the tibia by pairing a femoral component that is sized either up two sizes or down two sizes from a tibial component. A "size 3" CR femoral component may be used with any one of a "size 1, 2, 3, 4 or 5" tibial components (fixed or mobile), whereas a "size 1" CR femoral component may be used with any one of a "size 1, 2 or 3" tibial components (fixed or mobile). Similarly, a "size 5" fixed tibial component may be used with any one of a "size 3, 4, 5, 6 or 7" fixed insert (CR or PS). Using knee prosthesis system 100, each of these pairings allows optimally matching the femoral component to the insert to maintain desirable geometrical relationships.

Tables 1 lists the components of an exemplary embodiment of knee prosthesis system 100. Table 2 lists the femoral component sizes provided for each femoral component listed in Table 1. Table 2 also shows for each femoral component size the compatible insert size for each insert listed in Table 1 and the compatible tibial component sizes for each tibial component listed in Table 1.

**Table 1: Knee Prosthesis System Components**

| Component | No. of sizes | No. of thicknesses | No. of components |
|---|---|---|---|
| PS femoral (right) | 14 | --- | 14 |
| PS femoral (left) | 14 | --- | 14 |
| CR femoral (right) | 14 | --- | 14 |
| CR femoral (left) | 14 | --- | 14 |
| PS insert, mobile | 10 | 9 | 90 |
| PS insert, fixed | 10 | 9 | 90 |
| CR insert, mobile | 10 | 8 | 80 |
| CR insert, fixed | 10 | 8 | 80 |
| Tibial, mobile | 10 | --- | 10 |
| Tibial, fixed | 10 | --- | 10 |
| TOTAL | | | 416 |

**Table 2: Compatible Sizes**

| Femoral component size | Insert Size | Tibial component size |
|---|---|---|
| 1 | 1 | 1, 2, 3 |
| 2 | 2 | 1, 2, 3, 4 |
| 3 | 3 | 1, 2, 3, 4, 5 |
| 3N | 3 | 1, 2, 3, 4, 5 |
| 4 | 4 | 2, 3, 4, 5, 6 |
| 4N | 4 | 2, 3, 4, 5, 6 |
| 5 | 5 | 3, 4, 5, 6, 7 |
| 5N | 5 | 3, 4, 5, 6, 7 |
| 6 | 6 | 4, 5, 6, 7, 8 |
| 6N | 6 | 4, 5, 6, 7, 8 |
| 7 | 7 | 5, 6, 7, 8, 9 |
| 8 | 8 | 6, 7, 8, 9, 10 |
| 9 | 9 | 7, 8, 9, 10 |
| 10 | 10 | 8, 9, 10 |

The embodiment of knee prosthesis system 100 shown in Table 1 and Table 2 provides 2176 unique combinations of prosthesis components. In each of these combinations, the femoral component is distinctly-sized to fit the patient's femur while optimally matched to the insert, and the tibial component is distinctly-sized to fit the patient's tibia while compatible with the insert. As a result, knee prosthesis system 100 may allow surgeons to avoid compromising kinematic performance and life of the implanted joint for each patient of the worldwide patient population.

As previously noted, patella components may also be provided for implantation in combination with the knee prosthesis. The patella components may be provided in a plurality of sizes. Examples of patella implants that may be adapted for use in knee prosthesis system 100 are available under the trade mark PFC Sigma from DePuy Orthopaedics Inc. Another embodiment of knee prosthesis system 100 may also include two unique types of patella components, each type having five sizes, thereby allowing the surgeon to select from 21,760 unique combinations of components. In each of these combinations, the femoral component is distinctly-sized to match the patient's femur while optimally matched to the insert, and the tibial component is distinctly-sized to fit the patient's tibia while compatible with the insert.

Knee prosthesis system 100 allows the surgeon to select a combination of knee prosthesis components for implantation into the patient, in which the components are distinctly-sized to fit the femur and tibia of the patient, while also optimally matched to avoid compromising performance of the reconstructed joint. Knee prosthesis system 100 further provides PS femoral components that are proportionally sized to the femur since the PS insert (fixed or mobile) is matched to each PS femoral component. Knee prosthesis system 100 also may lower the cost and complexity of the necessary inventory of implant components to accommodate the worldwide patient population, due primarily to the interchangeability of the components.

As previously explained, there is a need for a knee prosthesis system that has mobile and fixed tibial components with stems of different lengths, but that does not require several different reaming instruments for preparing the tibia. As shown in Fig. 17, mobile tibial component 80 and fixed tibial component 70 may have an approximately similar or identical external size and configuration for each anatomical size, enabling the surgeon to prepare the proximal tibia in approximately the same way using the same instrumentation. This also allows the surgeon to implant either type of tibial component even after the proximal tibial has been surgically prepared.

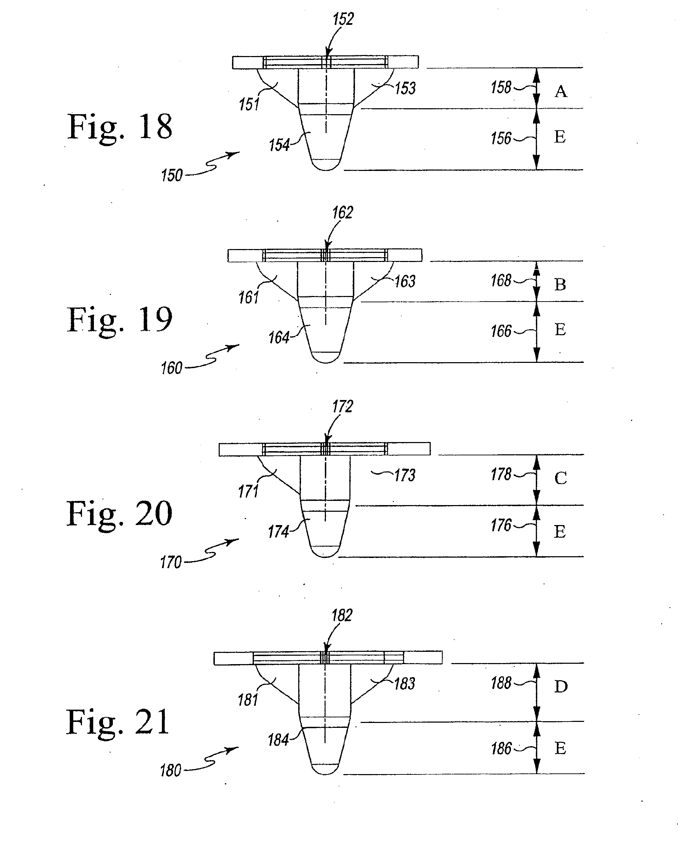

Figs. 18, 19, 20 and 21 show four representative sizes of mobile tibial component 80 of knee prosthesis system 100. Knee prosthesis system 100, for example, may have ten sizes of each of fixed tibial component 70 and mobile tibial component 80, as shown in Table 2. Fig. 18 is an anterior view of a size one, mobile tibial component 150 having a size one platform 152, a size one stem 154 and a pair of opposing keels 151, 153 extending between stem 154 and platform 152. Stem 154 has a distal portion 156 with a length "E" and a proximal portion 158 with a length "A".

Fig. 19 is an anterior view of a size three, mobile tibial component 160 having a size three platform 162, a size three stem 164 and a pair of opposing keels 161, 163. Stem 164 has a distal portion 166, also with length "E", and a proximal portion 168 with a length "B".

Fig. 20 is an anterior view of a size seven, mobile tibial component 170 having a size seven platform 172, a size three stem 174 and a pair of opposing keels 171, 173. Stem 174 has a distal portion 176, also with length "E", and a proximal portion 178 with a length "C".

Fig. 21 is an anterior view of a size nine, mobile tibial component 180 having a size nine platform 182, a size nine stem 184 and a pair of opposing keels 181, 182. Stem 184 has a distal portion 186, also with length "E", and a proximal portion 188 with a length "D".

As shown in Figs. 18, 19, 20 and 21, length "D" is greater than length "C", which is greater than length "B", which is greater than length "A". In general, increasing stem length corresponds to increasing length of the proximal portion of the stem, while the length of the distal portion remains constant.

Distal portions 156, 166, 176 and 186 may also have the same, generally conical shape. Proximal portions 158, 168, 178 and 188 may have approximately the same frustoconical shape and vary primarily in length. Keels 151, 153, 161, 163,171, 173, 181, 183 may have approximately similar configurations and orientations. As would be apparent to those skilled in the art, a surgeon may use the same reaming instrument to form a cavity to the desired depth in the proximal tibia to receive any one of the various sizes of stems 154, 164, 174 and 184. Because the external sizes and configurations of each of the plurality of distinctly-sized fixed tibial components may be approximately similar or identical to the corresponding one of the plurality of distinctly-sized mobile tibial components, the surgical preparation of the proximal tibia may be the same for a given size of either the fixed or mobile tibial components, and the required instrumentation may be the same for all sizes of both the fixed and mobile tibial components.

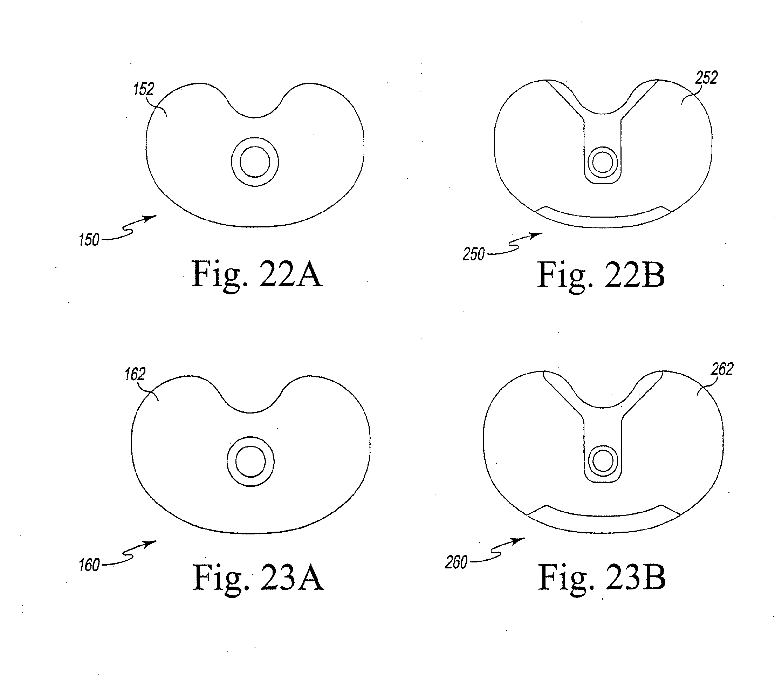

As previously explained, it is also desirable that the total knee replacement system have mobile and fixed tibial components with a common, platform profile or "footprint" that is optimized for coverage of the tibial plateau, interaction with surrounding tissues, kinematic performance and other factors. Figs. 22A, 22B, 23A, 23B, 24A, 24B, 25A and 25B show superior (plan) views of four representative sizes of fixed and mobile tibial components of knee prosthesis system 100. As noted previously, knee prosthesis system 100 may have ten sizes of each of fixed tibial component 70 and mobile tibial component 80, as shown in Table 2.

Fig. 22A shows size one mobile tibial component 150 to have a platform 152 that has a similar "footprint" or profile as a platform 252 of a size one fixed tibial component 250 shown in Fig. 22B.

Fig. 23A shows size three mobile tibial component 160 to have a platform 162 that has a similar profile as a platform 262 of a size one fixed tibial component 260 shown in Fig. 23B.

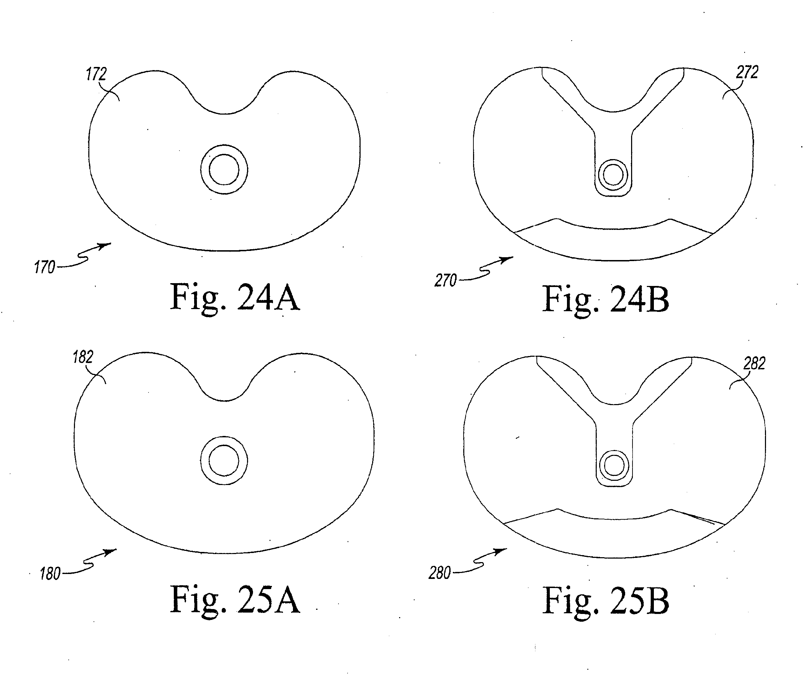

Fig. 24A shows size seven mobile tibial component 170 to have a platform 172 that has a similar profile as a platform 272 of a size one fixed tibial component 270 shown in Fig. 24B.

Fig. 25A shows size nine mobile tibial component 180 to have a platform 182 that has a similar profile as a platform 282 of a size one fixed tibial component 280 shown in Fig. 25B.

For each size of tibial component, the platform profile (as viewed from the top, in the direction of the stem axis) is the same for both mobile and fixed tibial components for a particular anatomical size. There is no need to change tibial component size to get the same, tibial plateau coverage when choosing between a mobile and a fixed prosthesis. Another benefit of the common platform shape is that the same casting tool or a portion of the tool may be used in the manufacture of both tibial components, enabling reduced component cost.

## Claims

1. A knee prosthesis system from which a surgeon may select a fixed or a mobile, total knee replacement prosthesis for implantation into a patient, the knee prosthesis system comprising:
a plurality of distinctly-sized fixed tibial components, each of which has a fixed platform defining a first, bone covering profile;
a plurality of distinctly-sized mobile tibial components, each of which has a mobile platform defining a second, bone covering profile;
in which the first and second, bone covering profiles are approximately the same for any one of the plurality of distinctly-sized fixed tibial components and a correspondingly-sized one of the plurality of distinctly-sized mobile tibial components.

2. The knee prosthesis system of claim 1, further comprising a plurality of distinctly-sized femoral components.

3. The knee prosthesis system of claim 2, further comprising a plurality of fixed inserts, each of which is sized and shaped for implantation between one of the plurality of distinctly-sized femoral components and at least one of the plurality of distinctly-sized fixed tibial components.

4. The knee prosthesis system of claim 2, further comprising a plurality of mobile inserts, each of which is sized and shaped for implantation between one of the plurality of distinctly-sized femoral components and at least one of the plurality of distinctly-sized mobile tibial components.

5. The knee prosthesis system of claim 1, in which:
each of the plurality of distinctly-sized fixed tibial components further includes a fixed stem extending distally from the fixed platform; and
each of the plurality of distinctly-sized mobile tibial components further includes a mobile stem extending distally from the mobile platform;
and in which the size and shape of the fixed stem of each of the plurality of distinctly-sized fixed tibial components is approximately the same as the size and shape of the mobile stem of a correspondingly-sized one of the plurality of distinctly-sized mobile tibial components.

6. The knee prosthesis system of claim 5, in which each of the fixed stems and each of the mobile stems include a distal portion having a distal length and a proximal portion having any one of a plurality of proximal lengths, such that each of the fixed stems and each of the mobile stems may have any one of a plurality of overall stem lengths.

7. The knee prosthesis system of claim 6, in which the distal portion of each of the fixed stems and the distal portion of each of the mobile stems has an approximately conical shape.

8. The knee prosthesis system of claim 6, in which the proximal portion of each of the fixed stems and the proximal portion of each of the mobile stems has an approximately frustoconical shape.

9. A knee prosthesis system from which a surgeon may select a fixed or mobile, total knee replacement prosthesis for implantation into a patient, the knee prosthesis system comprising:
a plurality of distinctly-sized fixed tibial components, each of which has a fixed platform defining a first, bone covering profile;
a plurality of distinctly-sized mobile tibial components, each of which has a mobile platform defining a second, bone covering profile;
a plurality of distinctly-sized femoral components;
a plurality of fixed inserts, each of which is sized and shaped for implantation between one of the plurality of distinctly-sized femoral components and at least one of the plurality of distinctly-sized fixed tibial components; and
a plurality of mobile inserts, each of which is sized and shaped for implantation between one of the plurality of distinctly-sized femoral components and at least one of the plurality of distinctly-sized mobile tibial components;
in which the first and second, bone covering profiles are approximately the same for any one of the plurality of distinctly-sized fixed tibial components and a correspondingly-sized one of the plurality of distinctly-sized mobile tibial components.

10. The knee prosthesis system of claim 9, in which:
each of the plurality of distinctly-sized fixed tibial components further includes a fixed stem extending distally from the fixed platform; and
each of the plurality of distinctly-sized mobile tibial components further includes a mobile stem extending distally from the mobile platform;
and in which the size and shape of the fixed stem of each of the plurality of distinctly-sized fixed tibial components is approximately the same as the size and shape of the mobile stem of a correspondingly-sized one of the plurality of distinctly-sized mobile tibial components.

11. The knee prosthesis system of claim 10, in which each of the fixed stems and each of the mobile stems include a distal portion having a distal length and a proximal portion having any one of a plurality of proximal lengths, such that each of the fixed stems and each of the mobile stems may have any one of a plurality of overall stem lengths.

12. The knee prosthesis system of claim 11, in which the distal portion of each of the fixed stems and the distal portion of each of the mobile stems has an approximately conical shape.

13. The knee prosthesis system of claim 11, in which the proximal portion of each of the fixed stems and the proximal portion of each of the mobile stems has an approximately frustoconical shape.
